# FASCICULE DE BREVET EUROPEEN

(11) **EP 2 854 914 B1**
(45) Date de publication et mention de la délivrance du brevet: **13.04.2016**
(21) Numéro de dépôt: 13739748.5
(22) Date de dépôt: 22.05.2013
(51) Int. Cl.: A61M 15/00

(54) **DISPOSITIF DE DISTRIBUTION DE PRODUIT FLUIDE.**
VORRICHTUNG ZUR AUSGABE EINES FLÜSSIGPRODUKTS
FLUID DISPENSER

(30) Priorité: 24.05.2012 FR 1254799
(43) Date de publication de la demande: 08.04.2015
(73) Titulaire: Aptar France SAS, 27110 Le Neubourg (FR)
(72) Inventeur: COLOMB, Arnaud, 78480 Verneuil Sur Seine (FR)
(74) Mandataire: CAPRI
(86) Numéro de dépôt international: PCT/FR2013/051111
(87) Numéro de publication internationale: WO 2013/175121

(56) Documents cités:
- WO-A2-2008/012458
- FR-A1- 2 660 550
- FR-A1- 2 936 425

## Description

La présente invention concerne un dispositif de distribution de produit fluide, et plus particulièrement un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. Un autre type d'inhalateur consiste à disposer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est d'abord chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a chargé la dose (soit à partir d'un réservoir multidoses, soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Un autre problème qui peut se poser concerne l'assemblage de certaines pièces, notamment mobiles, qui doivent supporter des contraintes importantes en fonctionnement, et pour lesquels l'assemblage doit être particulièrement fiable pour éviter tout risque de dysfonctionnement. Avec la petite taille de certaines pièces, il peut être compliqué de garantir une telle fiabilité d'assemblage. Le document WO 2008/012458 décrit un dispositif de l'art antérieur.

La présente invention a pour but de fournir un dispositif de distribution de produit fluide, en particulier un inhalateur de poudre sèche qui ne reproduit pas les inconvénients susmentionnés.

En particulier, la présente invention a pour but de fournir un tel dispositif qui soit simple et peu coûteux à fabriquer et à assembler, fiable à l'assemblage et en utilisation, garantissant une précision et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

L'objet de l'invention est défini par les revendications. La présente invention a donc pour objet un dispositif de distribution de produit fluide, comportant un corps, au moins un élément de capot monté pivotant sur ledit corps entre une position fermée et une position ouverte, ledit dispositif comportant au moins un réservoir individuel contenant une dose unique de produit fluide, tel que de la poudre, des moyens d'ouverture étant prévus pour ouvrir un réservoir individuel à chaque actionnement du dispositif, ledit dispositif comportant des moyens de support mobiles adaptés à déplacer un réservoir individuel contre lesdits moyens d'ouverture à chaque actionnement, lesdits moyens de support mobiles étant déplaçables entre une position de non-distribution et une position de distribution, lesdits moyens de support mobiles étant sollicités vers leur position de distribution par des moyens élastiques, tel qu'un ressort ou une lame élastique, et étant retenus en position de non-distribution par des moyens de blocage qui sont libérés par l'inhalation de l'utilisateur, ledit dispositif comportant un organe d'armement coopérant avec lesdits moyens élastiques et avec une surface de came, formée sur lesdits moyens de support mobiles, ledit organe d'armement et lesdits moyens élastiques étant assemblés dans un logement prévu dans une pièce de logement connectée à un élément de capot.

Avantageusement, ledit logement comporte de chaque coté des rails latéraux interrompus par des ouvertures latérales, ledit organe d'armement comportant des projections latérales adaptées à coopérer avec lesdites ouvertures latérales pour permettre l'assemblage dudit organe d'armement dans ledit logement, lesdits rails latéraux coopérant avec lesdites projections latérales dudit organe d'armement pour permettre un coulissement dudit organe d'armement dans ledit logement tout en maintenant ledit organe d'armement dans ledit logement.

Avantageusement, lesdites ouvertures latérales coopèrent avec lesdites projections latérales uniquement en position d'assemblage dudit organe d'armement, lesdits moyens élastiques sollicitant ledit organe d'armement hors de ladite position d'assemblage.

Avantageusement, lesdits moyens élastiques comportent un ressort à boudins disposé dans ledit logement.

Avantageusement, lesdits moyens de support mobiles supportent une roue de guidage, les réservoirs étant réalisés sous la forme d'une bande allongée comportant plusieurs réservoirs individuels disposés les uns à la suite des autres, ladite roue de guidage faisant avancer ladite bande à chaque actionnement du dispositif.

Avantageusement, lesdits moyens d'ouverture comportent un élément de perçage adaptés à découper une paroi de fermeture du réservoir de telle sorte que la ou les partie(s) découpée(s) n'obstrue(nt) pas la ou les ouverture(s) formée(s).

Avantageusement, le dispositif comporte un embout d'inhalation et un système de déclenchement par l'inhalation qui comporte une chambre d'air déformable coopérant avec ledit embout d'inhalation et un élément déclencheur coopérant avec ladite chambre d'air, de sorte que lors d'une inhalation à travers ledit embout d'inhalation, ladite chambre d'air est déformée et ledit élément déclencheur actionne lesdits moyens d'ouverture, de sorte qu'un réservoir est ouvert par lesdits moyens d'ouverture.

Ces caractéristiques et avantages et d'autres de la présente invention apparaîtront plus clairement au cours de la description détaillée suivante, faite en référence aux dessins joints, donnés à titre d'exemples non limitatifs, sur lesquels
- les figures 1 à 3 sont des vues schématiques en section transversale d'un dispositif de distribution selon un mode de réalisation avantageux de l'invention, respectivement avant ouverture, après ouverture mais avant inhalation, et après inhalation,
- les figures 4 et 5 représentent des vues schématiques en perspective, respectivement de dessus et de dessous, illustrant la pièce de logement,
- la figure 6 est une vue schématique en perspective de dessous de l'organe d'armement,
- la figure 7 est une vue schématique en perspective de dessus de la pièce de logement avec le ressort de chargement et l'organe d'armement assemblés dans le logement,
- les figures 8 et 9 représentent des vues schématiques en perspective, illustrant la phase d'assemblage de l'organe d'armement dans le logement de la pièce logement, respectivement avant et après assemblage, et
- les figures 10 à 13 sont des vues schématiques partielles en section transversale illustrant la phase d'assemblage de l'organe d'armement et du ressort de chargement dans le logement de la pièce logement.

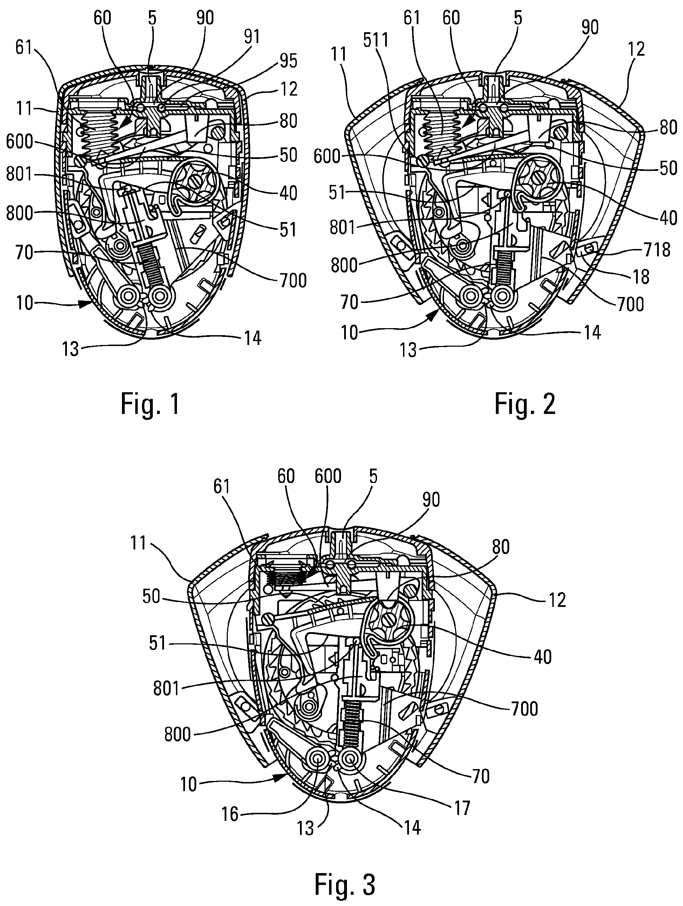

Sur les figures 1 à 3 est représenté un exemple avantageux d'un inhalateur de poudre sèche. Cet inhalateur comporte un corps 10 sur lequel peuvent être montées coulissantes deux parties 11, 12 formant capot adaptées à être ouvertes pour ouvrir et charger le dispositif. Le corps 10 peut être de forme environ arrondie comme représenté sur les figures, mais il pourrait avoir toute autre forme appropriée. Le corps 10 comporte un embout buccal ou d'inhalation 5 définissant un orifice de distribution à travers lequel l'utilisateur va inhaler lors de l'actionnement du dispositif. Cet orifice est typiquement disposé environ au centre de la partie supérieure du corps (dans la position représentée sur les dessins). Les capots 11, 12 peuvent s'ouvrir par pivotement autour d'un axe de rotation commun, ou autour de deux axes parallèles en étant engrené l'un avec l'autre. Tout autre moyen d'ouverture du dispositif est envisageable. En variante, le dispositif pourrait ne comporter qu'un seul capot au lieu de deux.

A l'intérieur du corps 10, il est prévu une bande de réservoirs individuels (non représentée dans des buts de clarté), également appelés blisters, réalisée sous forme d'une bande allongée sur laquelle les blisters sont disposés les uns derrière les autres, de manière connue. Cette bande de blisters est avantageusement constituée d'une couche ou paroi de base formant les cavités recevant les doses de poudre, et d'une couche ou paroi de fermeture qui obture de manière étanche chacun desdits blisters. Avant la première utilisation, la bande de blisters peut être enroulée à l'intérieur du corps 10, de préférence dans une partie de stockage, et des premiers moyens de déplacement de bande 40, notamment rotatifs, sont prévus pour progressivement dérouler et faire avancer cette bande de blisters. Des seconds moyens de déplacement 50, notamment pivotants sur le corps 10, sont prévus pour amener un blister respectif dans une position de distribution à chaque actionnement du dispositif. Ces seconds moyens de déplacement sont avantageusement pivotants entre une position de non-distribution et une position de distribution, dans laquelle un blister coopère avec lesdits moyens d'ouverture. La partie de bande comportant les blisters vides est avantageusement adaptée à s'enrouler dans un autre endroit dudit corps 10, de préférence une partie de réception, comme cela sera décrit plus en détail ci-après.

L'inhalateur comporte des moyens d'ouverture de blister 80 (seulement partiellement représentés dans des buts de clarté) comportant de préférence une aiguille de perçage et/ou de coupage de la couche de fermeture des blisters. De préférence, les moyens d'ouverture comportent un élément de perçage 80 fixe par rapport au corps 10, et contre lequel un blister respectif est déplacé à chaque actionnement par les seconds moyens de déplacement. Le blister est alors percé par ledit élément de perçage, qui pénètre dans ledit blister pour expulser la poudre au moyen du flux d'inhalation de l'utilisateur. Avantageusement, l'élément de perçage est adapté à découper une paroi de fermeture du réservoir de telle sorte que la ou les partie(s) découpée(s) n'obstrue(nt) pas la ou les ouverture(s) formée(s). Les documents WO 2006/079750 et WO 2009/007640 décrivent de tels moyens d'ouverture de blister, et sont donc intégrés dans la présente description à titre de références.

Les premiers moyens de déplacement 40 sont adaptés à faire avancer la bande de blisters après chaque inhalation de l'utilisateur. Les seconds moyens de déplacement 50 sont adaptés à déplacer le blister à vider contre lesdits moyens d'ouverture lors de l'actionnement, avant chaque inhalation. Ces seconds moyens de déplacement peuvent être sollicités par un élément élastique 70, tel qu'un ressort ou tout autre élément élastique équivalent, ledit élément élastique pouvant être pré-chargé lors de l'ouverture du dispositif. De préférence, les premiers moyens de déplacement 40 sont formés par une roue d'indexage qui reçoit et guide la bande de blisters. La suite de la description sera donc faite en référence à une telle roue d'indexage 40. Une rotation de cette roue d'indexage 40 fait avancer la bande de blisters. Avant chaque inhalation, un blister plein est toujours en position face aux moyens d'ouverture 80. Les seconds moyens de déplacement 50 peuvent comporter un organe pivotant autour d'un axe de rotation, ladite roue d'indexage 40 étant avantageusement montée rotative sur ledit organe pivotant.

Un cycle d'actionnement du dispositif peut être le suivant. Lors de l'ouverture du dispositif, les deux parties latérales 11, 12 formant capot sont écartées l'une de l'autre en pivotant sur le corps pour ouvrir le dispositif, et ainsi charger le dispositif. Dans cette position la roue d'indexage 40 ne peut pas se déplacer vers l'élément de perçage 80 car les seconds moyens de déplacement 50 sont retenus par des moyens de blocage appropriés (non représentés dans des buts de clarté). Les documents WO 2009/077700 et WO 2009/136098 décrivent de tels moyens de blocage, et sont donc intégrés dans la présente description à titre de références. Lors de l'inhalation par l'utilisateur à travers l'embout buccal, ces moyens de blocage sont débloqués, ce qui provoque alors le déplacement de ladite roue d'indexage 40 en direction de l'aiguille, et donc l'ouverture d'un blister.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture, on prévoit un système de déclenchement par l'inhalation 60 qui comporte avantageusement une chambre d'air 61 déformable sous l'effet de l'inhalation, cette chambre d'air étant adaptée à libérer les moyens de blocage. La chambre d'air 61 peut avantageusement être réalisée en forme de soufflet. L'inhalation de l'utilisateur provoque la déformation de ladite chambre d'air déformable, libérant ainsi lesdits moyens de blocage et permettant donc le déplacement des seconds moyens de déplacement, et donc d'un blister respectif, vers sa position d'ouverture. Le blister n'est donc ouvert qu'au moment de l'inhalation, de sorte qu'il est simultanément vidé. Il n'y a donc aucun risque de perte de dose entre l'ouverture du blister et son vidage.

L'inhalateur comporte en outre une chambre de distribution ou dispersion 90 qui est destinée à recevoir la dose de poudre après ouverture d'un blister respectif. Avantageusement, cette chambre de distribution est pourvue d'au moins une bille 91, de préférence plusieurs, qui se déplace(nt) à l'intérieur de ladite chambre 90 pendant l'inhalation, notamment pour améliorer la distribution du mélange air et poudre après ouverture d'un blister, afin d'augmenter l'efficacité du dispositif.

Il peut être avantageux que les moyens d'ouverture, en particulier l'élément de perçage, soient formés directement sur ladite chambre de distribution, par exemple à l'extrémité d'un canal 95 menant à ladite chambre 90.

Après l'inhalation, lorsque l'utilisateur referme le dispositif, tous les composants reviennent vers leurs positions de repos initiales. Le dispositif est alors prêt pour un nouveau cycle d'utilisation.

Selon un aspect avantageux de l'inhalateur, les blisters sont formés sur une bande allongée souple, qui, au début, est principalement stockée sous forme de bobine dans un logement de stockage à l'intérieur du corps 10 du dispositif. Avantageusement, cette bande de blisters enroulée est maintenue par des parois internes dudit logement de stockage sans que son extrémité arrière (dans le sens d'avancement de la bande de blisters) ne soit fixée par rapport audit corps 10, ce qui permet un assemblage plus aisé de cette bobine de bande de blisters à l'intérieur du dispositif. La bande de blisters est déplacée au moyen de la roue d'indexage 40 qui présente avantageusement au moins un, de préférence plusieurs évidements, dont la forme correspond à celle des blisters. Ainsi, lorsque cette roue d'indexage 40 tourne, elle fait avancer la bande de blisters. Bien entendu en variante ou de manière additionnelle, on pourrait utiliser d'autres moyens d'avancée de bande de blisters, par exemple en prévoyant un profil sur les bords longitudinaux latéraux de la bande de blisters, ledit profil étant adapté à coopérer avec des moyens d'entraînement appropriés. De même, des trous ménagés le long des bords latéraux de la bande de blisters pourraient également être utilisés pour faire avancer la bande de blisters au moyen de roues dentées coopérant avec lesdits trous.

Après ouverture d'un ou plusieurs blister(s), la partie de bande de blisters avec les blisters vidés doit pouvoir être stockée de manière aisée et non encombrante dans le dispositif, en évitant tout risque de blocage. Avantageusement, cette bande de blisters usée s'enroule automatiquement sur elle-même pour à nouveau former une bobine.

Selon encore un autre aspect de l'inhalateur, un dispositif de comptage ou d'indication de doses (non représenté dans des buts de clarté) est également prévu. Ce dispositif peut comporter des chiffres ou symboles inscrits directement sur la bande de blister et visibles à travers une fenêtre appropriée dans le corps 10 du dispositif. En variante, on pourrait envisager d'utiliser un compteur avec un ou plusieurs disque(s) ou anneau(x) rotatif(s) comportant des numéros ou symboles. Les documents WO 2008/012458 et WO 2011/154659 décrivent de tels compteurs, et sont donc intégrés dans la présente description à titre de références. Un but de l'invention est d'éviter de compter des doses qui ne sont pas distribuées, par exemple en cas d'erreur de manipulation, ou d'une manipulation incomplète du dispositif. Il est donc souhaitable que le compteur ou indicateur ne soir actionné qu'une fois que l'utilisateur a inhalé, puisque c'est cette inhalation qui conditionne l'ouverture et la distribution de la dose de chaque blister. Avantageusement, l'actionnement du compteur se fait donc après inhalation, lorsque l'utilisateur referme le dispositif.

Les figures 1 à 3 représentent un cycle d'ouverture et d'inhalation du dispositif.

L'élément de capot mobile 12 est solidaire d'un organe d'armement 800 qui peut coulisser dans un logement 710 approprié. Cet organe d'armement 800 est avantageusement pivotant par rapport audit corps 10 ensemble avec l'élément de capot 12. Cet organe d'armement 800 peut se déplacer contre le ressort 70, avantageusement un ressort à boudins, disposé dans ledit logement 710. L'organe d'armement 800 est donc connecté d'un côté audit ressort 70 et de l'autre côté, il coopère avec les seconds moyens de déplacement, en particulier avec un organe 50 pivotant sur le corps 10, et sur lequel est fixée de manière rotative la roue d'indexage 40.

Lorsque l'élément de capot mobile 12 est ouvert, comme représenté sur les figures 1 (position fermée) et 2 (position ouverte), l'organe d'armement 800 est déplacé dans son logement 710 en comprimant le ressort 70. L'organe pivotant 50 des seconds moyens de déplacement est lui empêché de se déplacer par les moyens de blocage susmentionnés, qui ne seront libérés qu'au moment de l'inhalation. Ainsi, en l'absence d'inhalation dans la position ouverte de la figure 2, la fermeture des éléments de capot 11, 12 provoquerait simplement le retour à la position de repos pour l'organe d'armement 800, et la décompression du ressort 70.

Ainsi, lorsque l'utilisateur ouvre l'inhalateur il charge le système (figures 1 & 2). S'il n'inhale pas et qu'il referme l'inhalateur, celui-ci reprend simplement sa position de départ sans déplacer la bande de blisters ni les moyens de blocage. Il n'y a donc aucun risque qu'un blister (et donc une dose de produit actif) soit perdu par un actionnement malencontreux ou incomplet dans lequel l'utilisateur n'exercerait pas d'inhalation entre l'ouverture et la fermeture.

L'ouverture du blister, son vidage, la distribution de la poudre dans les poumons de l'utilisateur, le déplacement de la bande de blisters pour amener un nouveau blister plein en face des moyens d'ouverture ainsi que le comptage de la dose ne sont donc possibles que si l'utilisateur inhale.

Les moyens de blocage, qui bloquent les seconds moyens de déplacement et notamment l'organe pivotant qui coopère avec l'organe d'armement, sont connectés à la chambre d'air déformable 61 sensible à l'inhalation de l'utilisateur, de sorte que lors de l'inhalation de l'utilisateur, ladite chambre d'air déformable se déforme, libérant lesdits moyens de blocage. Ceci permet le déplacement desdits seconds moyens de déplacement vers leur position de distribution sous l'effet de la force exercée par le ressort comprimé 70 sur l'organe d'armement 800 qui pousse sur l'organe pivotant 50. Ce déplacement provoque l'ouverture d'un blister plein et la distribution d'une dose.

Ledit élément de capot mobile 12 est connecté à un élément de logement 700, avantageusement par l'intermédiaire d'une ouverture 18, qui peut être de forme oblongue ou allongée, dans laquelle vient se placer un ergot ou similaire 718 dudit élément de logement 700.

Cet élément de logement 700 est avantageusement monté pivotant sur le corps 10 autour d'un axe de pivotement. Cet élément de logement 700 comporte un logement 710 qui reçoit le ressort 70. Ce ressort 70 coopère avec l'organe d'armement 800.

Une surface de came 51 est formée sur lesdits moyens de support mobiles 50, sur laquelle glisse l'organe d'armement 800. L'organe d'armement 800, lorsque l'élément de logement 700 est déplacé autour de son axe de pivotement lors du déplacement de l'élément de capot mobile 12, est donc adaptée à comprimer le ressort 70 lorsque l'élément de capot 12 est ouvert, et à décomprimer ledit ressort 70 lorsque ledit élément de capot 12 est refermé.

Avantageusement, l'organe d'armement 800 comporte, dans sa partie en contact avec la surface de came 51, une partie arrondie 801, telle qu'une extrémité en forme de boule, pour favoriser le glissement de l'organe d'armement 800 sur ladite surface de came 51.

Les moyens de support mobiles sont dans ce mode de réalisation réalisés sous la forme d'un organe 50 monté pivotant sur le corps 10 autour d'un axe de pivotement 511. La surface de came précitée 51 étant formée sur ledit organe pivotant 50, lorsque le ressort 70 est chargé lors de l'ouverture de l'élément de capot mobile 12, ledit organe pivotant 50 est sollicité vers sa position de distribution par ledit organe d'armement 800 et le ressort 70 comprimé.

Après l'inhalation, c'est-à-dire en position de distribution, les moyens de blocage ont été libérés et les moyens de support mobiles 50 ont été déplacés vers le haut par le ressort comprimé 70.

Par ailleurs, après inhalation et donc déplacement des moyens de support mobiles 50 vers la position de distribution, la fermeture de l'élément de capot mobile 12 ramène l'élément de logement 700 vers sa position de départ.

Avantageusement, les deux éléments de capot mobiles 11, 12 sont engrenés l'un dans l'autre via des engrenages appropriés 13, 14 pour garantir une ouverture et une fermeture symétrique desdits deux éléments de capot mobiles. Cet engrenage peut se faire à proximité de leur axe de pivotement 16, 17.

Selon l'invention, ledit organe d'armement 800 et lesdits moyens élastiques 70 sont assemblés dans ledit logement 710 prévu dans ladite pièce de logement 700 connectée à un élément de capot 12.

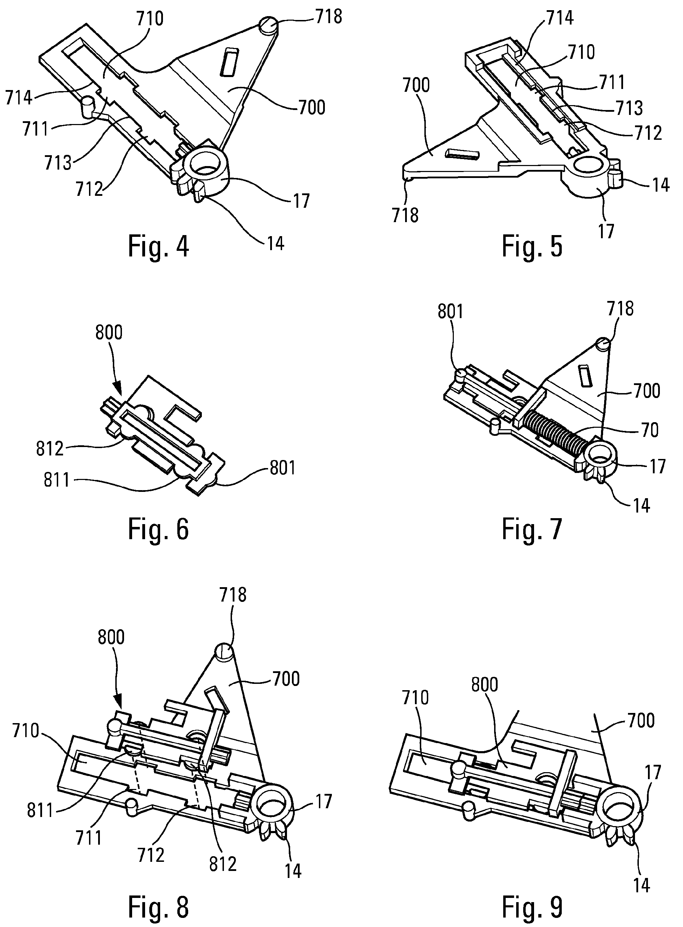

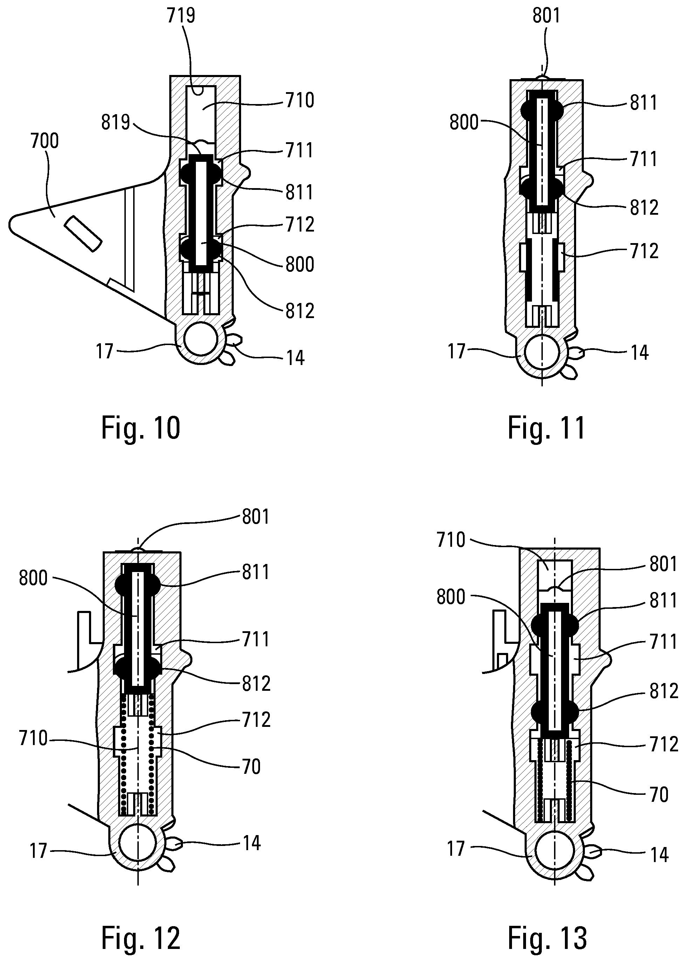

Avantageusement, ledit logement 710 comporte de chaque coté des rails latéraux 713, 714 interrompus par des ouvertures latérales 711, 712. L'organe d'armement 800 comporte des projections latérales 811, 812 adaptées à coopérer avec lesdites ouvertures latérales 711, 712 pour permettre l'assemblage dudit organe d'armement dans ledit logement 710. Ces rails latéraux 713, 714 coopèrent avec lesdites projections latérales 811, 812 dudit organe d'armement 800 pour permettre un coulissement dudit organe d'armement dans ledit logement 710 tout en maintenant ledit organe d'armement dans ledit logement. Lorsque l'organe d'armement 800 a ses projections latérales 811, 812 au niveau des ouvertures latérales 711, 712, il peut être inséré dans le logement 710, comme visible sur les figures 8 et 10. Dès que l'organe d'armement a coulissé dans ledit logement à partir de sa position d'insertion, comme visible sur les figures 11 à 13, lesdites projections latérales 811, 812 sont bloquées par lesdits rails latéraux 713, 714, ce qui permet un coulissement de l'organe d'armement dans le logement tout en empêchant cet organe d'armement de sortir dudit logement.

Avantageusement, lesdites ouvertures latérales 711, 712 du logement 710 coopèrent avec lesdites projections latérales 811, 812 de l'organe d'armement uniquement en position d'assemblage de l'organe d'armement, lesdits moyens élastiques 70 sollicitant ledit organe d'armement hors de ladite position d'assemblage. Ces moyens élastiques comprennent de préférence un ressort à boudin disposé dans ledit logement 710, comme visible sur les figures 7 et 11 à 13.

Les figures 10 à 13 illustrent un assemblage de l'organe d'armement 800 dans le logement 710. Comme expliqué ci-dessus, et comme visible sur les figures 8 et 10, l'organe d'armement 800 est placé au-dessus du logement 710 avec les projections latérales 811, 812 en face des ouvertures latérales 711, 712 du logement. L'organe d'armement peut alors être inséré dans le logement 710. Après son insertion, l'organe d'armement 800 est déplacé par coulissement dans le logement pour amener une extrémité avant 819 de l'organe d'armement 800 en butée avec un bord d'extrémité 719 du logement 710, comme visible sur la figure 11. Les moyens élastiques 70, en l'occurrence un ressort à boudin, sont alors insérés dans le logement 710 à l'état comprimé du coté opposé à ladite extrémité avant 819 de l'organe d'armement, et lorsque le ressort est libéré de sa condition comprimé, il va coopérer avec ledit organe d'armement 800 pour le solliciter élastiquement vers sa position en butée, comme visible sur la figure 12. Lorsque l'organe d'armement 800 coulissera dans le logement 710 en éloignement de sa position de butée, il va donc comprimer ledit ressort, ce qui permet d'assurer le fonctionnement du dispositif.

Avantageusement, après insertion du ressort dans le logement 710, l'organe d'armement 800 ne peut plus atteindre sa position d'insertion de la figure 10. La figure 13 montre la position où l'organe d'armement a été déplacé au maximum dans le logement, avec le ressort complètement comprimé. On constate que les projections latérales 811, 812 ne peuvent plus atteindre les ouvertures latérales 711, 712, et il n'y a donc aucun risque que l'organe d'armement puisse sortir du logement 710 après assemblage du ressort. Ceci garantit la fiabilité de fonctionnement du dispositif. La robustesse du dispositif est renforcée par l'absence de pièces collées, soudées ou clipsées pour assurer le maintient de l'organe d'armement dans le logement. Les forces créées par ce type de système d'armement et d'actionnement d'un inhalateur étant très importantes, cette notion de robustesse est particulièrement importante pour la fiabilité du dispositif. Par ailleurs, les pièces constitutives de ce système, en particulier l'organe d'armement 800 et la pièce de logement 700 avec son logement 710 sont simple à moulés, ce qui rend la fabrication et l'assemblage plus aisé et moins coûteux.

Dans tous les modes de réalisation décrits ci-dessus, la bande de blisters est formée par une bande présentant deux extrémités. En variante, on pourrait utiliser une bande continue. D'autres modifications sont également possibles sans sortir du cadre de la présente invention.

La présente invention permet donc de fournir un inhalateur de poudre sèche qui procure notamment les fonctions suivantes :
▪ une pluralité de doses individuelles de poudre stockées dans des blisters individuels étanches, par exemple 30 ou 60 doses stockées sur une bande enroulée en bobine ;
▪ la poudre libérée au moyen d'un perçage actionné par l'inhalation de l'utilisateur, ce perçage du blister étant réalisé au moyen d'un système de détection d'inhalation couplé à un système de libération pré-chargé ;
▪ des moyens d'entraînement de forme appropriée en prises avec les blisters pour réaliser le déplacement de la bande de blisters après chaque inhalation, et amener un nouveau blister plein dans une position dans laquelle il est destiné à être ouvert par les moyens d'ouverture appropriés ;
▪ des moyens pour éviter une perte de dose en cas d'ouverture de l'inhalateur mais en l'absence d'inhalation ;
▪ un indicateur de doses adapté à compter les doses seulement en cas d'inhalation.

D'autres fonctions sont également fournies par le dispositif de l'invention tel que cela a été décrit précédemment.

Il est à noter que les différentes fonctions, même si elles ont été représentées comme prévues simultanément sur l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les blisters individuels sont arrangés les uns par rapport aux autres, etc. Les moyens d'armement et le système de déclenchement par l'inhalation pourraient être réalisés différemment. Il en est de même des autres parties constitutives du dispositif.

Diverses modifications sont également possibles pour un homme du métier sans sortir du cadre de la présente invention tel que défini par les revendications annexées. En particulier, les diverses caractéristiques et fonctionnalités du dispositif décrites en référence aux dessins peuvent être combinées entre elles d'une quelconque façon appropriée.

## Revendications

1. Dispositif de distribution de produit fluide, comportant un corps (10), au moins un élément de capot (11, 12) monté pivotant sur ledit corps (10) entre une position fermée et une position ouverte, ledit dispositif comportant au moins un réservoir individuel contenant une dose unique de produit fluide, tel que de la poudre, des moyens d'ouverture (80) étant prévus pour ouvrir un réservoir individuel à chaque actionnement du dispositif, ledit dispositif comportant des moyens de support mobiles (50) adaptés à déplacer un réservoir individuel contre lesdits moyens d'ouverture (80) à chaque actionnement, lesdits moyens de support mobiles (50) étant déplaçables entre une position de non-distribution et une position de distribution, lesdits moyens de support mobiles (50) étant sollicités vers leur position de distribution par des moyens élastiques (70), tel qu'un ressort ou une lame élastique, et étant retenus en position de non-distribution par des moyens de blocage qui sont libérés par l'inhalation de l'utilisateur, ledit dispositif comportant un organe d'armement (800) coopérant avec lesdits moyens élastiques (70) et avec une surface de came (51), formée sur lesdits moyens de support mobiles (50), **caractérisé en ce que** ledit organe d'armement (800) et lesdits moyens élastiques (70) sont assemblés dans un logement (710) prévu dans une pièce de logement (700) connectée à un élément de capot (12), ledit logement comportant de chaque coté des rails latéraux (713, 714) interrompus par des ouvertures latérales (711, 712), ledit organe d'armement (800) comportant des projections latérales (811, 812) adaptées à coopérer avec lesdites ouvertures latérales (711, 712) pour permettre l'assemblage dudit organe d'armement dans ledit logement (710), lesdits rails latéraux (713, 714) coopérant avec lesdites projections latérales (811, 812) dudit organe d'armement pour permettre un coulissement dudit organe d'armement dans ledit logement (710) tout en maintenant ledit organe d'armement dans ledit logement.

2. Dispositif selon la revendication 1, dans lequel lesdites ouvertures latérales (711, 712) coopèrent avec lesdites projections latérales (811, 812) uniquement en position d'assemblage dudit organe d'armement, lesdits moyens élastiques (70) sollicitant ledit organe d'armement hors de ladite position d'assemblage.

3. Dispositif selon la revendication 2, dans lequel lesdits moyens élastiques (70) comportent un ressort à boudins disposé dans ledit logement (710).

4. Dispositif selon l'une quelconque des revendications précédentes, dans lequel lesdits moyens de support mobiles (50) supportent une roue de guidage (40), les réservoirs étant réalisés sous la forme d'une bande allongée comportant plusieurs réservoirs individuels disposés les uns à la suite des autres, ladite roue de guidage (40) faisant avancer ladite bande à chaque actionnement du dispositif.

5. Dispositif selon l'une quelconque des revendications précédentes, dans lequel lesdits moyens d'ouverture (80) comportent un élément de perçage adaptés à découper une paroi de fermeture du réservoir de telle sorte que la ou les partie(s) découpée(s) n'obstrue(nt) pas la ou les ouverture(s) formée(s).

6. Dispositif selon l'une quelconque des revendications précédentes, comportant un embout d'inhalation (5) et un système de déclenchement par l'inhalation (60) qui comporte une chambre d'air déformable (61) coopérant avec ledit embout d'inhalation (5) et un élément déclencheur (600) coopérant avec ladite chambre d'air (61), de sorte que lors d'une inhalation à travers ledit embout d'inhalation (5), ladite chambre d'air (61) est déformée et ledit élément déclencheur (600) actionne lesdits moyens d'ouverture (80), de sorte qu'un réservoir est ouvert par lesdits moyens d'ouverture.

## Patentansprüche

1. Ausgabevorrichtung für ein fluides Produkt, aufweisend einen Körper (10), mindestens ein Abdeckelement (11, 12), das auf dem Körper (10) zwischen einer geschlossenen Position und einer geöffneten Position schwenkbar montiert ist, wobei die Vorrichtung mindestens einen einzelnen Behälter aufweist, der eine einzige Dosis eines fluiden Produkts, wie ein Pulver, enthält, wobei Öffnungsmittel (80) vorgesehen sind, um bei jeder Betätigung der Vorrichtung einen einzelnen Behälter zu öffnen, wobei die Vorrichtung bewegliche Trägermittel (50) aufweist, die dazu geeignet sind, bei jeder Betätigung einen einzelnen Behälter gegen die Öffnungsmittel (80) zu verschieben, wobei die beweglichen Trägermittel (50) zwischen einer Nichtausgabeposition und einer Ausgabeposition verschiebbar sind, wobei die beweglichen Trägermittel (50) durch elastische Mittel (70), wie eine Feder oder ein elastisches Blatt, in ihre Ausgabeposition vorgespannt sind und durch Sperrmittel in der Nichtausgabeposition gehalten werden, die durch Inhalation des Benutzers freigesetzt werden, wobei die Vorrichtung eine Spanneinrichtung (800) aufweist, die mit den elastischen Mitteln (70) und mit einer Nockenfläche (51) zusammenwirkt, die auf den beweglichen Trägermitteln (50) gebildet ist, **dadurch gekennzeichnet, dass** die Spanneinrichtung (800) und die elastischen Mittel (70) in einer Aufnahme (710) aufgebaut sind, die in einem Aufnahmestück (700) vorgesehen ist, das mit einem Abdeckelement (12) verbunden ist, wobei die Aufnahme auf jeder Seite seitliche Schienen (713, 714) aufweist, die durch seitliche Öffnungen (711, 712) unterbrochen sind, wobei die Spanneinrichtung (800) seitliche Vorsprünge (811, 812) aufweist, die dazu geeignet sind, mit den seitlichen Öffnungen (711, 712) zusammenzuwirken, um den Aufbau der Spanneinrichtung in der Aufnahme (710) zu ermöglichen, wobei die seitlichen Schienen (713, 714) mit den seitlichen Vorsprüngen (811, 812) der Spanneinrichtung zusammenwirken, um ein Gleiten der Spanneinrichtung in der Aufnahme (710) zu ermöglichen und dabei die Spanneinrichtung in der Aufnahme zu halten.

2. Vorrichtung nach Anspruch 1, wobei die seitlichen Öffnungen (711, 712) nur in der Montageposition der Spanneinrichtung mit den seitlichen Vorsprüngen (811, 812) zusammenwirken, wobei die elastischen Mittel (70) die Spanneinrichtung außerhalb der Montageposition vorspannen.

3. Vorrichtung nach Anspruch 2, wobei die elastischen Mittel (70) eine Spiralfeder aufweisen, die in der Aufnahme (710) angeordnet ist.

4. Vorrichtung nach einem der vorhergehenden Ansprüche, wobei die beweglichen Trägermittel (50) ein Führungsrad (40) tragen, wobei die Behälter in Form eines länglichen Streifens ausgeführt sind, der mehrere einzelne Behälter aufweist, die nacheinander angeordnet sind, wobei das Führungsrad (40) bei jeder Betätigung der Vorrichtung den Streifen vorwärts bewegt.

5. Vorrichtung nach einem der vorhergehenden Ansprüche, wobei die Öffnungsmittel (80) ein Durchstoßelement aufweisen, das dazu geeignet ist, eine Verschlusswand des Behälters derart zu schneiden, dass der geschnittene Teil (die geschnittenen Teile) die gebildete(n) Öffnung(en) nicht behindert (behindern).

6. Vorrichtung nach einem der vorhergehenden Ansprüche, aufweisend einen Inhalationsansatz (5) und ein Auslösesystem durch Inhalation (60), das eine verformbare Luftkammer (61), die mit dem Inhalationsansatz (5) zusammenwirkt, und ein Auslöseelement (600) aufweist, das mit der Luftkammer (61) zusammenwirkt, so dass die Luftkammer (61) bei einer Inhalation durch den Inhalationsansatz (5) verformt wird und das Auslöseelement (600) die Öffnungsmittel (80) derart betätigt, dass ein Behälter durch die Öffnungsmittel geöffnet wird.

## Claims

1. A fluid dispenser device including a body (10) and at least one cover element (11, 12) that is mounted to pivot on said body (10) between a closed position and an open position, said device including at least one individual reservoir containing a single dose of fluid, such as powder, opening means (80) being provided for opening an individual reservoir each time the device is actuated, said device including movable support means (50) that are adapted to move an individual reservoir against said opening means (80) on each actuation, said movable support means (50) being displaceable between a non-dispensing position and a dispensing position, said movable support means (50) being urged towards their dispensing position by resilient means (70), such as a spring or a spring blade, and being held in their non-dispensing position by blocking means that are released by the user inhaling, said device including a cocking member (800) that co-operates with said resilient means (70) and with a cam surface (51) that is formed on said movable support means (50), the fluid dispenser device being **characterized in that** said cocking member (800) and said resilient means (70) being assembled in a housing (710) provided in a housing piece (700) connected to a cover element (12), said housing including side rails (713, 714) that are interrupted by side openings (711, 712), said cocking member (800) including side projections (811, 812) that are adapted to co-operate with said side openings (711, 712) so as to enable said cocking member to be assembled in said housing (710), said side rails (713, 714) co-operating with said side projections (811, 812) of said cocking member so as to enable said cocking member to slide in said housing (710), while holding said cocking member in said housing.

2. A device according to claim 1, wherein said side openings (711, 712) co-operate with said side projections (811, 812) solely in the assembly position of said cocking member, said resilient means (70) urging said cocking member out of said assembly position.

3. A device according to claim 2, wherein said resilient means (70) comprise a coil spring that is arranged in said housing (710).

4. A device according to any preceding claim, wherein said movable support means (50) support a guide wheel (40), the reservoirs being made in the form of an elongate strip comprising a plurality of individual reservoirs disposed one behind another, said guide wheel (40) causing said strip to advance each time the device is actuated.

5. A device according to any preceding claim, wherein said opening means (80) include a perforator element that is adapted to cut a closure wall of the reservoir in such a manner that the cut portion(s) does/do not obstruct the opening(s) that is/are formed.

6. A device according to any preceding claim, including an inhalation piece (5) and an inhalation trigger system (60) that comprises a deformable air chamber (61) that co-operates with said inhalation piece (5), and a trigger element (600) that co-operates with said air chamber (61), such that during inhalation through said inhalation piece (5), said air chamber (61) is deformed and said trigger element (600) actuates said opening means (80), such that a reservoir is opened by said opening means.
